# FASCICULE DE BREVET EUROPEEN

(11) **EP 0 501 081 B1**
(45) Date de publication et mention de la délivrance du brevet: **15.05.1996**
(21) Numéro de dépôt: 91420068.8
(22) Date de dépôt: 28.02.1991
(51) Int. Cl.: A61B 17/00, A61M 5/00

(54) **Appareil destiné à la destruction des varices des membres inférieurs chez l'homme**
Vorrichtung zum Zerstören von Krampfadern
Apparatus for the destruction of varicose veins

(43) Date de publication de la demande: 02.09.1992
(73) Titulaire: Cadet, Pierre, F-26000 Valence (FR)
(72) Inventeur: Cadet, Pierre, F-26000 Valence (FR)

(56) Documents cités:
- EP-A- 0 358 999
- US-A- 1 644 919
- US-A- 4 418 693

## Description

La présente invention concerne un dispositif destiné à détruire les varices des membres inferieurs chez l'homme.

Le dispositif selon l'invention permet de remédier aux inconvénients des méthodes employées actuellement ç'est à dire le stripping , la sclérose faite a l'aide d'une seringue et d'une aiguille , la cryochirurgie ainsi qu' à ceux d'une méthode utilisant une seringue et une sonde . Cette dernière a été pratiquée de façon publique dès I909 jusqu'à son abandon en I948. Elle a fait également l'objet d'une demande européenne EP 358 999 . Lors de son utilisation on introduit dans la veine un tube flexible ouvert à une extrémité pour l'injection par seringue d'un produit sclérosant et fermé à l'autre extrémité mais comportant au voisinage de celle ci un trou latéral dans la paroi du tube pour permettre au liquide sclérosant de ressortir du tube et d'entrer en contact avec les varices . Cette technique est très dangereuse puisque le produit sclérosant est introduit par seringue sous pression dans la veine et risque de passer directement , rapidement et sans aucun contrôle dans la voie veineuse profonde ou dans la voie artérielle par des communications artério-veineuses et de provoquer une sclérose brutale des artéres pouvant nécessiter l'amputation de tout ou d'une partie du membre inférieur.

La présente invention concerne donc un dispositif pour détruire les varices des membres inférieurs chez i'homme comportant un tube en matière plastique caractérisé en ce que ledit tube est ouvert aux deux extrémités et en ce qu'un fil de nylon sur lequel se trouvent deux renflements , l'un au milieu du fil agissant comme piston dans le tube,l'autre à une extrémité de ce fil agissant comme bouchon sur l'extrémité B du tube. Cette extrémité est hérissée sur sa face extérieure de 6 petites aspérités .

Le dispositif comporte :
Un tube en matière plastique semi rigide (1) que nous appellerons"tube" et qui présente à l'extérieur de l'une de ses extrémités 6 petites aspérités (3) d'un millimétre chacune et dirigées vers l'autre extrémité du tube. Les aspérités sont faites de la matière même du tube et réparties circulairement autour de celui-ci.

Un fil de nylon (2) au milieu duquel se trouve un renflement cylindrique (5) dont le diamétre extérieur est égal au diamétre intérieur du tube (1) Ce renflement appelé "piston" .La partie supérieure de ce piston étant biseautée pour permettre une entrée plus facile du piston dans le tube (1)par l'orifice B. A l'extrémité de ce fil se trouve un renflement qu'on peut diviser en deux parties : une première partie dont le diamétre extérieur est égal au diamétre intérieur du tube , deforme cylindrique et biseautée comme le piston ; la deuxième partie qui fait suite à la première a une forme de poire d'un diamètre un peu plus grand que celui de la première partie .La première partie en pénétrant dans l'extrémité B du tube servira de bouchon à celui-ci .La seconde partie en forme de poire facilitera le cheminement du tube dans la varice d'une part et sera facilement maintenu au travers de la peau au niveau de la cheville au moment du retrait du tube.

L'extrémité C du fil de nylon sera introduite dans le tube par l' extrémité où se trouvent les aspérités B . Lorsque l'extrémité du fil sortira par l'extrémité A du tube , le piston (5) sera près de l'orifice B;
En tirant sur le fil on fera pénétrer le piston dans B. Quand la partie inférieure du piston (5) arrivera au niveau del'orifice B du tube on mettra l'extrémité B du tube dans un récipient contenant un liquide sclérosant . A ce moment on tire sur le fil de nylon , le piston (5) monte dans le tube et entraine avec lui le liquide sclérosant .Puis la partie cylindrique du bouchon (4) arrivera au niveau de B et ayant été biseautée pénétrera facilement dans cet orifice . La partie en forme de poire viendra buter sur le tube qui se trouvera ainsi parfaitement fermé à sa partie inférieure.

Le dispositif est alors prêt à être utilisé.

L'intervention se fera en deux phases :
1 - Ouverture au niveau du pli de l'aine et ablation de la crosse de la saphène dite crossectomie.
2 - Par l'extrémité béante de la saphène on introduit le dispositif extrémité B du tube en premier et on pousse ainsi le dispositif pour amener le renflement D au niveau de la cheville . La direction des aspérités ne géne pas la progression du tube.

A ce moment on pose une bande élastique de la racine des orteils jusqu'en haut de la cuisse . La varice est bien vidée de son sang et celui-ci n'y reviendra pas puisque la crosse de la saphène est enlevée.

L'opérateur saisit alors entre deux doigts au travers de la peau , à la cheville, la partie en poire et renflée du bouchon (4).De l'autre main il prend la partie A du tube (1) et la tire en dehors de la varice tout droit ou en lui imprimant de légers mouvements de rotation.

L'intima est éraillée par les aspérités.

Le liquide sclérosant est déposé d'une façon parfaitement continue , régulière et sans aucune pression car le liquide est déposé et prend la place laissée libre par le tube en se retirant.

Le fil de nylon est ensuite retiré, et la forme du bouchon (4) augmente la pénétration du liquide sclérosant dans les éraillures dues aux aspérités.

Le dispositif selon l'invention est particuliérement destiné à la destruction des varices des membres inférieurs chez l'homme.

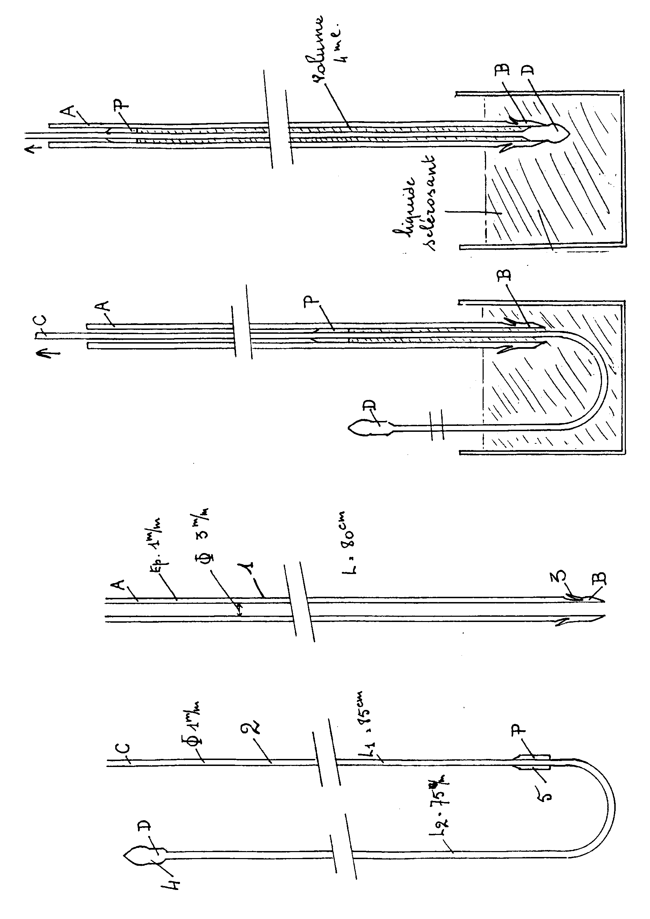

## Revendications

1. Dispositif pour détruire à l'aide d'un liquide sclérosant lesvarices des membres inférieurs chez l' homme comportant un tube (1)en matière plastique caractérisé en ce que ledit tube (1)est ouvert aux deux extrémités et en ce qu'un fil (2)de nylon sur lequel se trouvent deux renflements (4 et5), l'un (5) au milieu du fil (2) agissant comme piston dans le tube (1) , l'autre (4) à une extrémité de ce fil (2) agissant comme bouchon sur l'extrémité du tube(1) et en ce que l'une (B)des extrémités ( A , B ) du tube (1) est hérissée sur sa face exterieure de 6 petites aspérités (3).

2. Dispositif selon la revendication 1 caractérisé en ce que les aspérités à l'extrémité du tube (1) sont réparties réguliérement autour du tube.

3. Dispositif selon la revendication 2 caractérisé en ce que les aspérités à l'extrémité du tube (1) sont taillées dans la matière même du tube.

4. Dispositif selon l'une des revendications 2 ou 3 caractérisé en ce que les aspérités sont toutes dirigées dans le même sens , ç'est à dire vers l'autre extrémité du tube (1) .

5. Dispositif selon l'une des revendications précédentes caractérisé en ce que le renflement (5) au milieu du fil est cylindrique et en ce que son diamétre est égal au diamétre intérieur du tube (1)

6. Dispositif selon l'une des revendications précédentes caractérisé en ce que le renflement (5) qui se trouve au milieu du fil (2) et appelé piston présente une extrémité supérieure biseautée pour faciliter sa pénétration dans l'orifice du tube (1) en B .

7. Dispositif selon l'une des revendications précédentes caractérisé en ce que le renflement bouchon (4) situé à l'extrémité du fil (2) comportr une partie cylindrique et une partie en forme de poire .

8. Dispositif selon la revendication 7 caractérisé en ce que la partie cylindrique du renflement bouchon (4) est biseautée à sa partie supérieure pour mieux pénétrer dans le tube (1) pur l orifice B.

9. Dispositif selon l'une des revendications 7 ou 8 caractérisé en ce que la partie du renflement bouchon (4) située à l'extrémité du fil et qui fait suite à la partie cylindrique a un diamétre légérement supérieur à celui de la partie cylindrique.

10. Dispositif selon l'une des revendications 7 à 9 caractérisé en ce que la partie en forme de poire permet une introduction plus facile dans la varice.

## Claims

1. A device for destroying varices in the lower members of a human being with a sclerosing liquid, comprising a plastic tube (1) characterised in that said tube (1) is open at both extremities and in that a nylon thread (2) bears two swellings ((4) and (5)), one (5) at the middle of the thread acts as a piston in tube (1), the other (4) at the end of thread (2) acts as a stopper on the extremity of tube (1) and in that one (B) of the extremities (A, B) of tube (1) is bristling with 6 small asperities (3) on its external face.

2. A device according to claim 1 characterised in that the asperities at the extremity of tube (1) are evenly distributed around the tube.

3. A device according to claim 2 characterised in that the asperities at the extremity of tube (1) are carved in the very material of the tube.

4. A device according to any of claims 2 or 3 characterised in that the asperities are all orientated towards the same direction, i.e. towards the other extremity of tube (1).

5. A device according to any of the preceding claims characterised in that the swelling (5) in the middle of the thread is cylindrical and in that its diameter is equal to the internal diameter of tube (1).

6. A device according to any of the preceding claims characterised in that the swelling (5) in the middle of the thread (2) and called piston exhibits a chamfered upper extremity in order to facilitate its penetration into the aperture of tube (1) in B.

7. A device according to any of the preceding claims characterised in that the swelling acting as a stopper (4) located at the extremity of thread (2) comprises a cylindrical part and a pear-shaped part.

8. A device according to claim 7 characterised in that the cylindrical part of the swelling acting as a stopper (4) is chamfered in its upper part in order to better penetrate into tube (1) through aperture B.

9. A device according to any of claims 7 or 8 characterised in that the part of the swelling acting as a stopper (4) located at the extremity of the thread and which follows the cylindrical part has a diameter slightly bigger than the diameter of the cylindrical part.

10. A device according to any of claims 7 to 9 characterised in that the pear-shaped part facilitates the introduction into the varix.

## Patentansprüche

1. Eine Vorrichtung zur Zerstörung von Krampfadern in den unteren Gliedmaßen eines Menschen mit Einbeziehung einer skleroszierenden Flüssigkeit enthaltend ein Rohr (1) aus Kunststoff dadurch gekennzeichnet, daß das Rohr (1) an den beiden Enden geöffnet ist und einen Nylonfaden (2) enthält, der mit zwei Anschwellungen (4) und (5) versehen ist, wobei eine (5) in der Mitte des Fadens als Kolben im Rohr (1) wirkt und die andere (4) an dem Ende des Fadens (2) als Stöpsel auf das Ende des Rohres (1) wirkt und daß eines (B) der Enden (A,B) des Rohres (1) mit 6 kleinen Unebenheiten auf seiner externen Fläche bespickt ist.

2. Eine Vorrichtung nach Anspruch 1 dadurch gekennzeichnet, daß die Unebenheiten auf dem Ende des Rohres (1) regelmässig um das Rohr verteilt sind.

3. Eine Vorrichtung nach Anspruch 2 dadurch gekennzeichnet, daß die Unebenheiten auf dem Ende des Rohres (1) in das Rohrmaterial selbst geschliffen sind.

4. Eine Vorrichtung gemäß einem der Ansprüche 2 oder 3 dadurch gekennzeichnet, daß die Unebenheiten alle nach der gleichen Richtung orientiert sind, d.h. nach dem anderen Ende des Rohres (1) zeigen.

5. Eine Vorrichtung gemäß einem der obigen Ansprüche dadurch gekennzeichnet, daß die Anschwellung (5) in der Mitte des Fadens zylindrisch ist und daß ihr Durchmesser mit dem inneren Durchmesser des Rohres (1) gleich ist.

6. Eine Vorrichtung gemäß einem der obigen Ansprüche dadurch gekennzeichnet, daß die als Kolben genannte Anschwellung (5) in der Mitte des Fadens (2) ein zur Erleichterung ihrer Penetration in der Öffnung des Rohres (1) abgekantetes oberes Ende besitzt.

7. Eine Vorrichtung gemäß einem der obigen Ansprüche dadurch gekennzeichnet, daß die als Stöpsel wirkende Anschwellung (4) am Ende des Fadens (2) einen zylindrischen Teil und einen birnenförmigen Teil besitzt.

8. Eine Vorrichtung gemäß Anspruch 7 dadurch gekennzeichnet, daß der zylindrische Teil der als Stöpsel wirkenden Anschwellung (4) in seinem oberen Teil abgekantet ist, um besser in das Rohr (1) penetrieren zu können.

9. Eine Vorrichtung gemäß einem der Ansprüche 7 oder 8 dadurch gekennzeichnet, daß der Teil der als Stöpsel wirkenden Anschwellung (4), der sich am Ende des Fadens befindet und dem zylindrischen Teil folgt, einen Durchmesser aufweist, der etwas grösser ist als der Durchmesser des zylindrischen Teils.

10. Eine Vorrichtung gemäß einem der Ansprüche 7 bis 9 dadurch gekennzeichnet, daß der birnenförmige Teil eine leichtere Penetration in der Krampfader ermöglicht.
